# EUROPEAN PATENT APPLICATION

(11) **EP 1 174 089 A1**
(43) Date of publication of application: **23.01.2002**
(21) Application number: 00202628.4
(22) Date of filing: 21.07.2000
(51) Int. Cl.: A61B 17/11

(54) **Vascular anastomic bonding device**

(71) Applicant: Technologiestichting STW, 3502 GA Utrecht (NL)
(72) Inventor: Scheltes, Julien Serge, 2517 BG Den Haag (NL); Hejkens,Martijn, 2512 JR Den Haag (NL); Pistecky, Peter Vladimir, 3121 TB Schiedam (NL); Gründemann, Paul Frederik, 1055 TG Amsterdam (NL)
(74) Representative: Jorritsma, Ruurd

(57) **Abstract**

The invention relates to a vascular anastomotic device (1, 20, 49) comprising a ring-shaped frame (2, 21, 50) and a plurality of hook elements (4, 22, 51) extending from said ring-shaped frame, which hook elements are movable between an introduction position located relatively closely to a centre line (6, 23, 57) of the device, and a fixation position, in which the hook elements are located at a larger distance from the centre line of the device. The bonding device is characterised in that the hook elements comprise a gripper part (7, 35, 52) with a pointed end (9, 25, 59), the gripper part extending on one side of the ring-shaped frame (2, 21, 50), radially inwardly or downwardly in an axial direction, and an actuator part (3, 26, 28; 54, 55) attached to the gripper part, extending on a side of the ring-shaped frame (2, 21, 50), radially outwardly or in an axial direction, the actuator part (3, 26, 28; 54, 55) being movable with respect to the frame, wherein the gripper parts (7, 35, 52) can be displaced radially outwardly by movement of the actuator parts.

The extra-luminal vascular anastomotic bonding device allows coronary artery bypass grafting allowing full vision on the anastomotic line, while using relatively simple tools. The device can be attached to the graft with minimal eversion and avoids overstretching and excessive compressive forces.

## Description

### Technical field of the invention

The invention relates to a vascular anastomotic device comprising a ring-shaped frame and a plurality of hook elements extending from said ring-shaped frame, which hook elements are movable between an introduction position located relatively closely to a centre line of the device, and a fixation position, in which the hook elements are located at a larger distance from the centre line of the device.

### Background of the invention

Coronary artery bypass grafting (CABG) procedures may be carried out using minimally invasive techniques in which blood vessels are joined via special tools through a small opening in the thorax. An overview of the development of CABG is given in US patent nr. 5,868,763.

A bypass is made by connecting a bypass graft artery or vein through a side-to-end, side-to-side or end-to-end anastomosis to the obstructed coronary artery, distally from the obstruction. The connection between the graft vessel and the arteriotomy edges in the coronary artery is conventionally carried out by suturing, which has the advantage of maintaining good visibility on the anastomotic line by the use of slender instruments, of establishing intima-intima apposition (contact of the inner vessel walls) and of enabling the creation of the anastomosis at variable angles between the graft and the arteriotomy. However, the problem of obtaining a proper apposition is not solved by suturing as the proper positioning of the individual sutures, which are not larger than a few mm, for obtaining proper apposition, requires great surgical skills. Using steel clips or staples instead of a running suture or individual sutures results in a proper fixation, but the regularity of the anastomosis is still greatly dependent on the skills of the surgeon and some pressure necrosis due to the clips may occur.

In order to apply all staples or clips around the anastomotic line in one action, end-to-side staplers are known comprising an anvil with pots to guide the plastic deformation of the staples, a cartridge holding the staples before application, and a driver pushing the staples out of the cartridge against the anvil. The known application tools are complex. Although the apposition problem is solved using such so-called one-shot devices, and good intima-intima contact is only possible by everting the graft over the anvil, but the use of the anvil creates excessive strain in both graft and recipient artery, and the visibility is poor due to the bulky application tool. Fixation of obtained apposition requires large fixation forces, necessary to deform the staples and clips, and may interfere with maintaining a proper well apposition. Misplacement of bonding elements can occur, and the compression force at the bonding location cannot be controlled, such that the bonding elements might be applied too loose.

When the bonding elements establishing the connection between the graft and recipient vessel are connected to a frame, the cartridge can be omitted and the application tool can be simplified. A one component bonding device according to the preamble of claim 1 is known from US patent nr 5,366,462. In the known bonding device, a ring-shaped frame carries two sets of axially protruding hook elements, which are introduced into the graft and into the arteriotomy respectively, whereafter the hooks are deformed by a relatively simple and slender application tool. A disadvantage of the known extra-luminal bonding device is that it is located near or against the anastomotic line and hence reduces its visibility, rendering application difficult. In the known device, the anastomotic line may be slackened or buckled upon deformation of the hook elements, resulting in a poor connection and potential leakage along the anastomic line. Finally, the known extra-luminal bonding device may result in excessive squeezing of the graft and recipient artery upon the connection, which may lead to damage of the vessel wall, leakage or even wall necrosis.

### Objects of the invention

It is an object of the present invention to provide a extra-luminal vascular anastomotic device which allows a clear vision on the anastomic line and which can be applied with simple, preferably conventional instruments.

It is a further object of the present invention to provide an extra-luminal bonding device which is of a relatively simple design and which can be attached to the graft with minimal eversion of the graft wall and hence minimal damage due to overstretching.

It is another object of the present invention to provide an extra-luminal bonding device, which results in a proper, and leakage free connection of the graft to the recipient vessel along a substantially regular and smooth anastomotic line.

It is another object of the present invention to provide an extra-luminal bonding device which can be, with the graft mounted thereon in the introduction position, easily placed within the arteriotomy without a forced widening thereof.

It is a further object of the present invention to provide an extra-luminal bonding device, which is suitable to be applied by automatic applicator tools.

It is another object of the present invention to provide a bonding device which can applied to form a substantially trumpet-shaped transition of graft end to anastomosis.

It is finally an object of the present invention to provide an extra-luminal bonding device, which avoids exertion of excessive compressive forces along the anastomotic line, and in which the hook elements can be indepently applied by a surgeon, to tailor the connection to the situation during surgery.

### Summary of the invention

The above objects are achieved by a vascular anastomotic bonding device which is characterised in that the hook elements comprise a gripper part having a pointed end, the gripper part extending on one side of the ring-shaped frame, radially inwardly or downwardly in an axial direction, and an actuator part, attached to the gripper part, extending on a second side of the ring-shaped frame, radially outwardly or in an axial direction, the actuator part being movable with respect to the frame, wherein the gripper parts can be displaced radially outwardly by movement of the actuator parts.

The graft may be easily placed around the pointed ends of the gripper parts of the anastomotic device without undue eversion thereof. The pointed ends may be straight, and can be bent into a V-shaped form after mounting of the graft on the device. In another embodiment, the pointed end has a generally V-shaped configuration defined by a first leg of the gripper part, and a second leg extending transversely to the first leg. Next, the gripper parts can be easily introduced into the arteriotomy without forced stretching thereof. When the graft is properly aligned with respect to the recipient vessel, the actuator parts can be deflected, such that the gripper parts are displaced radially outwardly and the hook elements are stretching the anastomotic line. During the connection operation of the graft to the recipient vessel, which may be carried out with simple and conventional tools such as forceps and pincers, the view on the anastomotic line is not obscured as the ring-shaped frame, the gripper parts and the actuator parts do not overlap with the anastomotic line. Upon connection of the graft, the vessel walls are properly apposed in a preferably non-squeezing manner such that no damage to the vessel walls occurs. Upon the radially displacing the gripper parts, the anastomotic line is stretched such that a leakage free connection is achieved. Intima to intima, rim to rim or overlap contact is established. Due to the radial movement of the hook shaped parts, the wall of the recipient artery is penetrated by the pointed ends, creating a bonding that solves the fixation problem. The dimensions of the vascular anastomotic device of the present invention can be easily adjusted to fit the size of the graft, and the application tool to operate the actuator part can be designed such that it can easily be used with a range of bonding devices.

The term "ring-shaped frame" as used herein is intended to comprise circular, oval, square and other shapes of frame having a generally closed contour around a central aperture.

In one embodiment, the gripper parts extend in radial direction from the ring-shaped frame towards a centreline of the ring. The actuator parts extend radially outwardly from the ring-shaped frame and are with their proximal ends connected with the ring-shaped frame. The actuator parts have the form of a plate-shaped lever to which the ends of the gripper parts are connected. By lifting the plate-shaped levers the connection parts, which are in the introduction position oriented substantially in the plane of the ring-shaped frame, are rotated vertically downwardly and radially outwardly around the frame, such that the pointed ends will penetrate the wall of the recipient vessel, thus creating a solid bonding. Due to the radially outward movement of the gripper parts, the anastomosis will be trumpet-shaped, which is favourable for a good flow profile and which minimises intimal hyperplasia.

The ring-shaped frame will be located outside the lumen above the anastomotic line, at a distance corresponding to the height by which the gripper parts projects below the ring-shaped frame after rotating the actuator parts to assume the fixation position placing the frame outside of the lumen results in minimisation of the thrombus surface and in prevention of narrowing of the lumen due to intra-luminal structures. Furthermore, location of the ring-shaped frame above the anastomotic line results in good visibility of the anastomotic line during every stage of the construction of the anastomosis.

The plate-shaped levers of the actuator parts can be gripped with straightforward conventional pincers and needle holders. The ease of operation of the actuator parts will result in simplified design of an automated applicator suitable in use for closed chest, beating heart by-pass grafting. As the gripper parts can be individually actuated by bending the actuator part upwardly, it is possible to tailor the degree of deflection of each gripper part to the local situation of the anastomosis. Minimal squeezing action of the vessel walls is achieved, thus preventing pressure necrosis.

In another embodiment of the vascular anastomotic bonding device according to the present invention, the gripper part extends from the pointed end located near the centre line of the ring-shaped frame, to an end position, radially outwardly of the ring-shaped frame. The gripper parts are slidably connected to the ring-shaped frame so as to be displaceable in a radial direction. The actuator part comprises an abutment member extending at or near the end position of the gripper part, substantially transversely thereto. A spring means is compressed between the ring-shaped frame and the abutment member, for forcing the gripper parts radially outwardly.

The flat surface of the ring-shaped frame can be placed on the heart surface, at a radial distance around the anastomotis site to maintain a good view of the anastomotic line. To keep the gripper parts in the introduction position, a restraining element may be applied around the free ends of the gripper parts to compress the spring means and to slide the pointed ends of the gripper parts to a location close to the axial centre line of the ring-shaped frame. Removal of the restraining element will result in the spring means expanding and retracting of the gripper parts away from the centre line of the frame and interconnection of the graft and the recipient vessel.

Another embodiment comprises gripper parts extending in a generally axial direction from the ring-shaped frame, a driver ring and actuator ring, interconnected by axial bars, being slidably mounted on the ring-shaped frame to engage with the gripper parts to deflect the gripper parts in the connection position. With this embodiment, a solid bonding of the graft to the recipient vessel can be achieved and a trumpet-shaped anastomosis can be formed. As the walls of both the graft and the recipient artery will be compressed, the gaps between the bonding locations are closed whereby leakage is prevented.

In a further embodiment of an anastomotic device according to the present invention, the hook elements are made of resilient material which, when unrestricted, returns to its fixation position. This embodiment provides for radial movement of the hooks to return to their equilibrium position upon connection of the graft to the recipient vessel due to the spring forces of the hooks and stretching of the anastomotic line. The hook elements can also move in a tangential direction for obtaining equal stretching of each segment of the anastomotic line. Hereby overstretching of one segment due to misplacement of a hook can be prevented. Preferably, the hook elements are substantially V-shaped such that upon placement of the graft thereon, the graft is properly supported by the hooks and does not easily slide off the gripper parts. The pointed end of the V-shaped hooks can also, on radial displacement of the gripper parts be pointed upwards to penetrate the wall of the recipient vessel.

### Brief description of the drawings.

Some embodiments of a vascular anastomotic bonding device according to the present invention will be described in detail with reference to the non-limiting drawings. In the drawings:
Fig. 1 shows a plan bottom view of a first embodiment of the vascular anastomotic bonding device according to the present invention, comprising actuator parts in the form of radially extending levers.
Fig. 2 and 3 show cross-sectional. views of the bonding device of fig. 1 in the introduction position and in the fixation position, respectively.
Figures 4a-4c and fig. 4d show the different stages of operation of the bonding device of fig. 1, in a longitudinal and in a transverse cross-sectional view respectively,
Fig. 5 shows a second embodiment of the bonding device according to the present invention comprising actuator and a driver rings for radially expanding the hook elements,
Figures 6a-6c show the different stages of the operation of the bonding device of fig. 5,
Fig. 7 and 8 show a third embodiment of the bonding device, comprising resilient hook elements,
Fig. 9 shows a plan bottom view of a fourth embodiment of the bonding device according of the present invention comprising radially displaceable hook element biased by spring elements,
Fig. 10 shows a cross-sectional view of the bonding device of fig. 9, and
Fig. 11 shows an embodiment of a bonding device of the type of fig. 5, without an actuator ring.

### Detailed description of the invention

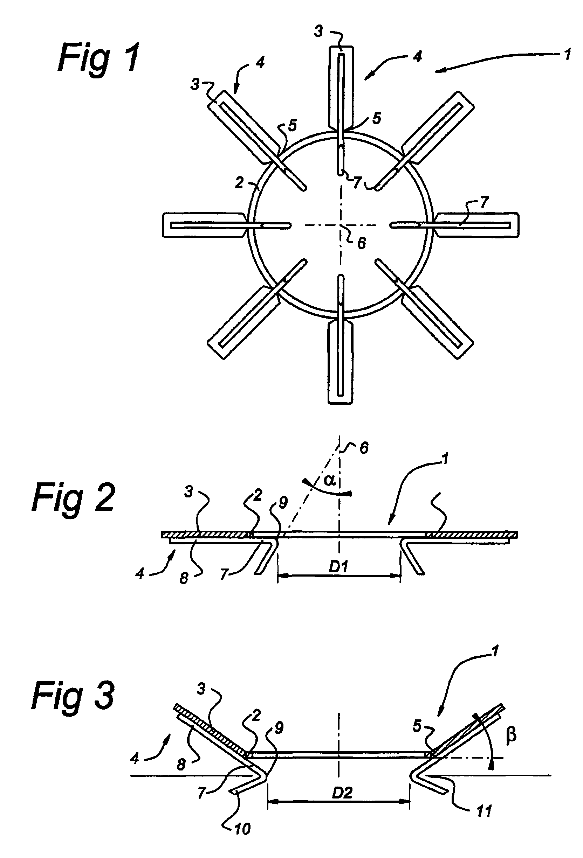

Fig. 1 shows the extra-luminal bonding device 1 comprising a ring-shaped metal frame 2. Around the outer circumferential edge of the ring-shaped frame 2, hook elements 4 are connected comprising bendable levers 3, which carry on their lower sides pointed gripper parts 7 extend towards the centreline 6 of the frame 2. At the point of connections of the levers 3 to the ring 2, the levers 3 are of reduced width for facilitating bending of the levers 3.

As can be seen from fig. 2, the hook elements 4 comprise gripper parts 7 that are formed by a bar 8 that is connected to the lower surface of the levers 3, the bar 8 having a pointed end 9. The pointed end 9 is formed by a transverse arm 10 extending at an acute angle with respect to the bar 8 such as to result in a generally V-shaped point. With "generally V- shaped" as used herein, it is meant that the arm 10 can extend at an angle of between 90 ° and substantially 0 ° with respect to the bar 8.

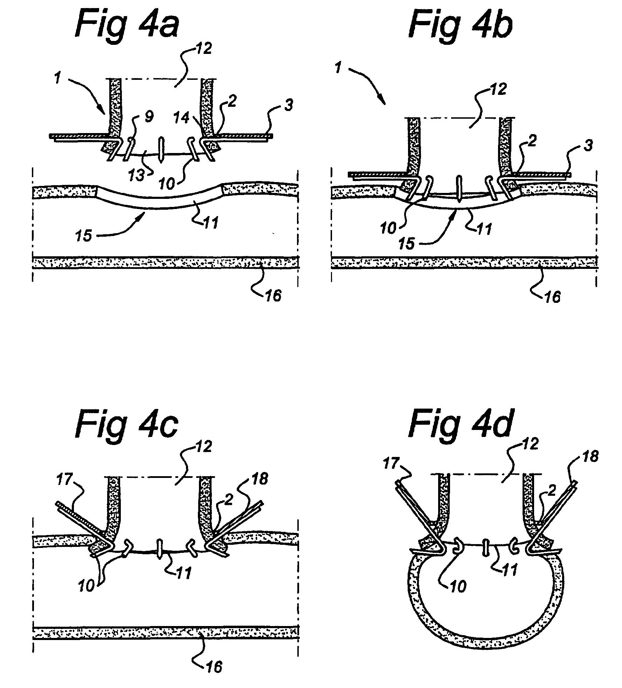

In the configuration that is shown in fig. 2, the bonding device 1 is in its introduction position in which the diameter D1, defined by the pointed ends 9, is relatively small. In this position, the proximal end part of a graft may be introduced through the ring-shaped frame 2 to extend with it is lower edge below the pointed ends 9. Upon slightly retracting the graft, the pointed ends 9 can puncture the vessel wall of the graft and (see fig. 4a) can then be introduced from the outside of the graft wall to extend to an intra-luminal position thereof. Upon the introduction of the pointed ends 9 into the arteriotomy, the levers 3 are bent upwards with respect to the ring-shaped frame 2 through an angle β of for instance 60°, such as shown in fig. 3, whereby the gripper parts 7 are placed in their fixation positions. The diameter D2 is increased, and the transverse arms 10 are radially translated and rotated though an angle α of about 60°, such as to extend substantially horizontally and puncture the recipient vessel wall near the anastomotic line 11.

Fig. 4a shows a prepared graft vessel 12 which has be mounted on the bonding device 1 of the present invention. The proximal edge 13 of the graft 12 is lowered through the central opening of the ring -shaped frame 2 to extend below the pointed ends 9. Then, the graft 12 is pulled back such that the points at the end of arms 10 penetrate the vessel wall to form apertures 14 and to extend from the outer surface to the inner surface of the graft 12. Next, the arteriotomy 15 is made in recipient vessel 16. The arteriotomy 15 in the present embodiment is shown to be oval, but could also be of other shape, such as for instance circular.

As shown in fig. 4b, the mounted graft 12 is lowered into the arteriotomy 15 During this operation, levers 17 and 18 may be bent upwardly as shown in fig. 4c, prior to introduction, to facilitate mounting of the graft 12 into the arteriotomy 15 of the recipient vessel 16.

As is shown in the transverse cross-sectional view of fig. 4d, All the levers 3 of the hook elements 4 are bent upward such that the arms 10 penetrate the vessel wall of recipient vessel 16 from an intra-luminal position to an extra-luminal position. This anchors the anastomosis and by the compression resulting of the stretching of the anastomotic line 11 due to the increased diameter D2 of the bonding device 1 in its fixation position, leakage is prevented.

Upon placing the bonding device 1 in the fixation position as shown in fig. 3 and fig 4d, the pointed ends 9 and the arms 10 are rotated. In the introduction position shown in fig. 2 and in fig. 4a and 4b, the arms 10 extend at an angle α with respect to the centre line 6, of approximately 60°. In the fixation position shown in fig. 3 and fig. 4d, the levers 3 and bars 8 have been rotated about an angle β of about 60 ° such that the arms 10 extend approximately perpendicular to the centre line 6. This rotation has as an advantage that the graft 12 can be mounted without overstretching the proximal end 13. The pointed ends 9 can with the arms 10 in the position shown in fig. 2, fig. 4a and fig. 4b be introduced into the arteriotomy 15 without causing a forced widing thereof. Finally, the horizontal orientation of the arms 10 in the fixation position will result in,solid bonding to the recipient vessel 16 due to easy penetration of the wall thereof, by the pointed ends of the arms 10.

The wall of proximal end part 13 of the graft 12 will, after mounting on the bonding device 1, slide to the comers formed at the points of connection of arms 10 to bars 8. Upon lifting of the levers 3, the pointed ends 9 are translated from a first, smaller diameter D1 to a second, larger diameter D2 together with the wall of the recipient vessel 16, after introduction of the mounted graft into the anastomosis 15, such as shown in fig. 4c. This causes the anastomotic line 11 to be stretched, which has as an advantage that the anastomosis will be trumpet-shaped as shown in fig. 4d. Such a trumpetshape is favorable for a good flowprofile and minimises intimal hyperplasia. Furthermore, by stretching of the anastomotic line 11, the recipient vessel 16 will be compressed, which closes the gaps between the bonding locations. The degree of compression can be adjusted by the surgeon by controlling the angle β through which the levers 3 are lifted.

As can be seen in fig. 4a-4d, the bonding device 1 is located outside the lumen of recipient vessel 16, which has as an advantage that the thrombus forming surface is minimised and that no narrowing of the lumen due to intra-luminal structures will occur. Furthermore, the location of the bonding device 1 above the anastomotic line 11 warrants clear visibility of the anastomotic site during every step of the construction of the anastomosis shown in fig. 4a-4d, which will give the surgeon full control of the bonding device.

Tools for applying the bonding device according to the present invention can be pincers and needle holders with which the surgeon is well acquainted and which require little, if any additional training. Due to the slender instrument tips of the known tools, a good view of the anastomotic site during construction of the anastomosis is possible at all times.

Finally, as the movements which are needed to mount the bonding device 1 are relatively simple and straightforward, an automated applicator will be easy to develop for use in closed chest, beating heart bypass grafting operations.

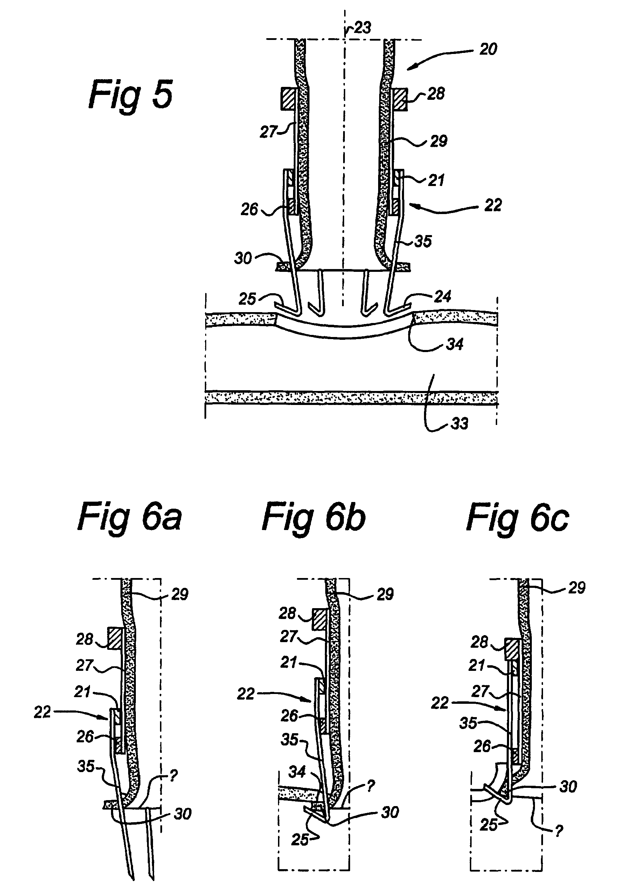

Fig. 5 shows an extra-luminal bonding device 20 in which a number of hook elements 22 comprise flexible bars 35 that extend generally in the axial direction of the longitudinal centre line 23, at radially distributed positions around the frame ring 21. Transverse arms 24 at the end of bars 35 extend generally perpendicular to the longitudinal centre line 23 in a radial direction, and form pointed ends 25 on the hook elements 22. An extender ring 26 is connected via axial bars 27 to an actuator ring 28. By pushing down on the actuator ring 28, or by pulling the frame ring 21 upward, the extender ring 26 slides along the flexible bars 35 of hook elements 22 such that these bars are expanded in a radial direction. A graft 29 has been mounted on the bonding device 20, wherein the pointed ends 25 of the transverse arm 24 have been introduced trough the proximal end 30 of the graft 29.

Fig. 6a shows the bonding device 20 carrying the graft 29, in the introduction position, wherein the actuator ring 28 is at its upmost position and the extender ring 26 abuts against the ring-shaped frame 21. When the hook elements 22 are introduced through the proximal end 30 of the graft 29, the pointed ends 25 are in line with the axial bars 22 as shown in fig. 6a.

The graft 29 is shoved upwardly in the axial direction to free the ends 25 of the hooks 22 which then can thereafter be bent form the pointed transverse arms 24 that are located closely together in the introduction position shown in fig. 6b, before placing the hook elements 22 within the arteriomoty 31 of receiving vessel 33 such as shown in fig. 6b.

Thereafter, the frame ring 21 can be pushed upwards towards the actuator ring 28 such that the flexible bars 35 slide along the extender ring 26 to radially expand the bars 35 and to push the pointed ends 25 radially outwardly, as is shown in fig. 6c.

As shown in fig. 6c, the extender ring 26 is placed in the arteriomotomy 31 and the wall of the recipient artery 33 is penetrated by the pointed ends 25, whereby the anastomosis is completed.

The bars 35 of the hook elements 22 are made of steel wires which are very flexible, such that the pointed ends 25 can be easily moved towards each other to assume a small diameter of the introduction configuration.

In addition to obtaining the same advantages as have been mentioned with respect to the extra-luminal bonding device 1 shown in figures 1-4d, the bonding device 20 provides ease of movability of the bars 35 in a tangential direction. This results in an equal stretch distribution over all segments of the anastomotic line 34. Furthermore, the visibility of the anastomotic line 34 is increased by being able to push the proximal end 30 of the graft upwards along the flexible bars 35.

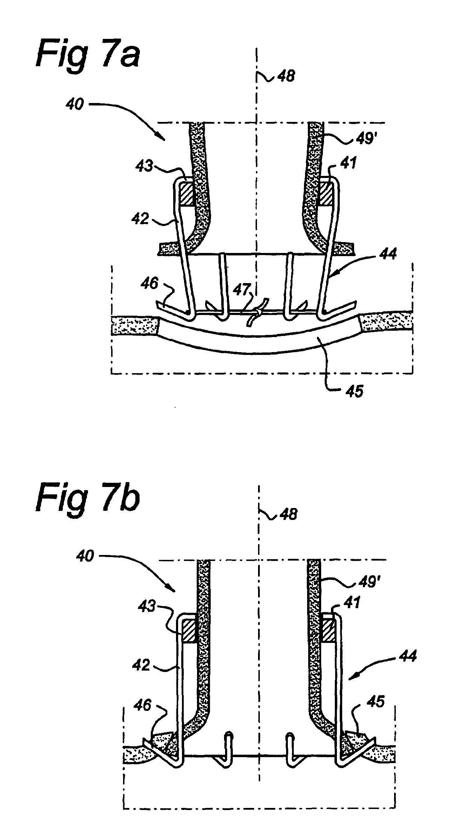

Fig. 7 shows an embodiment of a bonding device 40 in which at spaced-apart positions along the circumference of a ring-shaped frame 41, the bars 42 of a number of resilient hook elements 44 are connected to the frame 41 via base plates 43. The material of the hook elements has elastic properties, such as to assume a radially expanded equilibrium position when unrestrained. By use of a simple restraining wire 47, the bars 42 of the hook elements 44 can be held in the introduction position The graft 49' can be mounted on the bonding device 40 and the pointed ends 46 can be placed in the ateriotomy of the recipient vessel, below the anastomotic line 45. Upon removal of the restraining element 47, the hook elements 44 will expend radially, connecting the graft 49' to the recipient vessel. The bonding device 40 is of a very simple design and can be manufactured without interacting components which are subject to friction or high tolerances. The simple bonding device 40 can be applied with application tools of reduced complexity. Furthermore, the very open design of the bonding device 40 results in very good visibility of the anastomotic line 45. The hook elements 42 can easily be moved in radial direction as well as in a tangential direction, which results in an equal stretching of anastomotic line 45, thus avoiding overstretching of segments of the anastomotic line 45 due to wrong placement of a hook element 44.

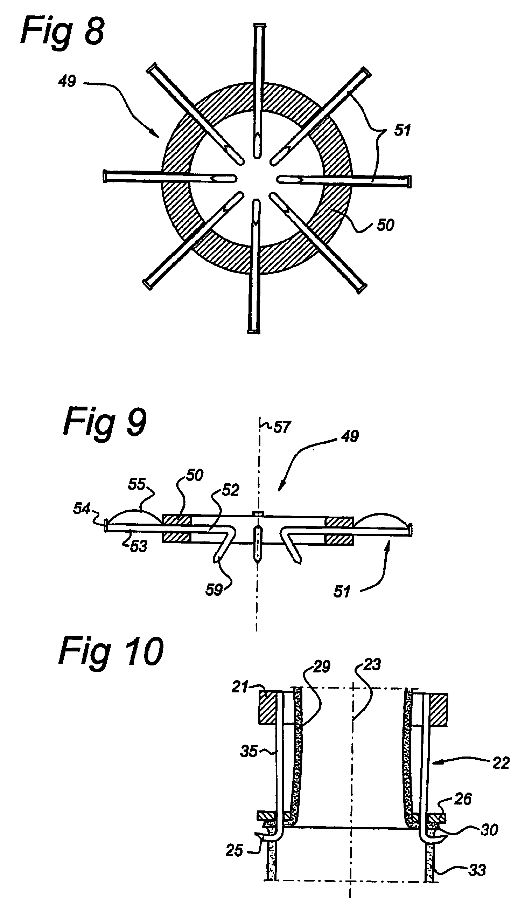

As has been shown in fig. 9, a ring-shaped frame 50 of a further embodiment of a bonding device 49 carries a number of radially displaceable hook elements 51, which can slide a radial direction with respect to the frame 50. The hook elements 51 comprise a gripper part 52 having a first leg 59 and transverse arm 56 as shown in fig. 10, and an actuator part 53 having an abutment member 54. Between the abutment member 54 and the ring-shaped frame 50, a spring element 55 is placed which pushes the pointed end 59 of the hook element 52 radially outwardly towards the inner edge of the ring-shaped frame 50. By placing a compressive restraining element such as wire loop, around the abutment members 54, the pointed ends 59 can be moved towards the longitudinal centre line 57 to place the bonding device 49 into the introduction position. After attaching the graft and placing the pointed ends 59 into the arteriotomy, the restraining element may be removed such that due to expansion of the springs 55, the pointed ends 59 are moved radially away from the centre line 57 and the anastomotic line is stretched. This flat device 50 can be placed on the heart surface to improve the view of the anastomotic line and provides a very compact bonding device with minimal dimensions in the axial direction. A leakage free connection between graft and recipient vessel can be obtained in a non-squeezing manner.

Fig. 10 shows an embodiment wherein a flat extender ring 26 is slidably mounted on the hook elements 22. Upon sliding extender ring 26 downward, the hook elements are forced radially outwardly, such that the pointed ends penetrate the wall of recipient vessel 33. In the fixation position that is shown in fig. 11, the extender ring 26 and the pointed ends of hook elements 22 provide a leakage free squeezing contact of the proximal end 30 of the graft 29 with the wall of the recipient vessel 33.

## Claims

**1.** Vascular anastomotic device (1, 20, 49) comprising a ring-shaped frame (2, 21, 50) and a plurality of hook elements (4, 22, 51) extending from said ring-shaped frame, which hook elements are movable between an introduction position located relatively closely to a centre line (6, 23, 57) of the device, and a fixation position, in which the hook elements are located at a larger distance from the centre line of the device, **characterised in that** the hook elements comprise a gripper part (7, 35, 52) with a pointed end (9, 25, 59), the gripper part extending on one side of the ring-shaped frame (2, 21, 50), radially inwardly or downwardly in an axial direction, and an actuator part (3, 26, 28; 54, 55) attached to the gripper part, extending on a side of the ring-shaped frame (2, 21, 50), radially outwardly or in an axial direction, the actuator part (3, 26, 28; 54, 55) being movable with respect to the frame, wherein the gripper parts (7, 35, 52) can be displaced radially outwardly by movement of the actuator parts.

**2.** Vascular anastomotic device (1, 20, 49) according to claim 1, the gripper part having a generally V-shaped pointed end (9, 25, 59) defined by a first leg (8, 35, 52) and a second leg (10, 25, 56) extending transversely to the first leg (8, 35, 52).

**3.** Vascular anastomotic device (1, 49) according to claim 1 or 2, wherein in the introduction position, the gripper parts (7) extend in a radial direction from the ring-shaped frame (2) towards a centre line (6) of the ring-shaped frame (2), the actuator parts (3) extending along substantially the same radial directions as the gripper parts (7), radially outwardly from the ring-shaped frame (2), the actuator parts (3) being with a proximal end (5) connected to the ring-shaped frame.

**4.** Vascular anastomotic device (1) according to claim 3, the actuator parts comprising a plate-shaped lever (3), attached with its proximal end to the ring-shaped frame (2), the gripper parts (7) comprising a bar (8) extending from the pointed end (9) located near the centre line (6) of the ring-shaped frame (2), to a position on the plate-shaped lever (3), radially outwardly of the ring-shaped frame (2), the bar (8) being connected to the lever (3).

**5.** Vascular anastomotic device (1) according to claim 4, **characterised in that,** the lever (3) at its point of connection (5) to the ring, is of reduced width.

**6.** Vascular anastomotic device (49) according to claim 1 or 2, **characterised in that,** the gripper part (52) extends from the pointed end (59) located near the centre line (57) of the ring-shaped frame (50), to an end position, radially outwardly of the ring-shaped frame (50), the gripper part (52) being slidably connected to the ring-shaped frame (50) so as to be slidable in a radial direction, the actuator part comprising an abutment member (54) extending at or near the end position of the gripper part, substantially transversely thereto, and a spring means (55) being compressed between the ring-shaped frame (50) and the abutment member, for forcing the gripper part (52) radially outwardly.

**8.** Vascular anastomotic device (20) according to claim 1 or 2, **characterised in that** the gripper parts (22) extend in a generally axial direction on a downward side of the ring-shaped frame (21), the actuator part comprising a driving ring (26), engaging the gripper parts (22), and being slidably connected to the ring-shaped frame (21) or to the gripper parts (22).

**9.** Vascular anastomotic device (20) according to claim 8, **characterised in that**, the driving ring (26) is located on a proximal side of the ring-shaped frame (21), the actuator part comprising an actuator ring (28), located on a distal side of the ring-shaped frame (21), the driving ring (26) and the actuator ring (28) being interconnected via axial bars (27) that are slidable along the ring-shaped frame (21).

**10.** Vascular anastomotic device (20) according to claim 9, the outer diameter of the driving ring (26) and of the actuator ring (28) being equal to, or larger than the inner diameter of the ring-shaped frame (21).

**11.** Vascular anastomotic device (20) according to claim 8, **characterised in that the** driving ring (26) comprises a flat ring having apertures extending around the gripper parts (22).

**12.** Vascular anastomotic device (40), comprising a ring-shaped frame (41) and a plurality of hook elements (44) extending from an outer circumferential edge of said ring-shaped frame (41), which hook elements comprise a generally axially orientated part (42), and are movable between an introduction position located relatively closely to a centre line (48) of the device, and a fixation position, in which the hook elements (44) are located at a larger distance from the centre line of the device, **characterised in that** the hook elements comprise a resilient material which, when unrestricted, returns the axially oriented parts (42) to their fixation positions, the hook elements (44) having a substantially V-shaped pointed end (46), defined by a first leg and a second leg extending transversely to the first leg.

**13.** Vascular anastomotic device (40) according to claim 12, **characterised in that,** the hook elements (44) comprise a flexible bar, and a plate-shaped base (43) connected to the ring shaped frame.
